# EUROPÄISCHE PATENTSCHRIFT

(11) **EP 1 511 787 B1**
(45) Veröffentlichungstag und Bekanntmachung des Hinweises auf die Patenterteilung: **09.11.2005**
(21) Anmeldenummer: 03755043.1
(22) Anmeldetag: 21.05.2003
(51) Int. Cl.: C08G 63/12, C08G 18/42

(54) **VERFAHREN ZUR HERSTELLUNG VON POLYESTERPOLYOLEN MEHRWERTIGER ALKOHOLE**
METHOD FOR PRODUCING POLYESTERPOLYOLS OF POLYVALENT ALCOHOLS
PROCEDE DE PRODUCTION DE POLYOLS DE POLYESTERS A PARTIR D'ALCOOLS POLYVALENTS

(30) Priorität: 24.05.2002 DE 10223055
(43) Veröffentlichungstag der Anmeldung: 09.03.2005
(73) Patentinhaber: BASF Aktiengesellschaft, 67056 Ludwigshafen (DE)
(72) Erfinder: WARTINI, Alexander, 67245 Lambsheim (DE); DERNBACH, Matthias, 69221 Dossenheim (DE); TISCHER, Gerlinde, 01945 Ruhland (DE); SIRCH, Tilman, 67105 Schifferstadt (DE)
(86) Internationale Anmeldenummer: PCT/EP2003/005307
(87) Internationale Veröffentlichungsnummer: WO 2003/099902

(56) Entgegenhaltungen:
- EP-A- 0 338 540
- EP-A- 0 807 617
- EP-A- 0 908 481
- EP-A- 0 970 995
- WO-A-01/47847
- WO-A-97/16401
- DE-A- 10 029 055
- GB-A- 1 290 036
- US-A- 3 097 245
- US-A- 6 018 074
- US-A- 6 096 905
- US-B1- 6 187 971

## Beschreibung

Die vorliegende Erfindung beschreibt ein Verfahren zur Herstellung von Polyesterpolyolen mehrwertiger Alkohole durch einfache oder mehrfache Veresterung einer Dicarbonsäure und/oder einem Anhydrid einer Dicarbonsäure mit den entsprechenden mehrwertigen Alkoholen in gegebenenfalls einem Lösungsmittel und gegebenenfalls unter Zusatz eines sauren Katalysators unter Abtrennung des Reaktionswassers.

Unter mehrwertigen Alkoholen werden dabei solche Verbindungen verstanden, die mehrere Hydroxygruppen aufweisen, beispielsweise 2 bis 6, bevorzugt 2 bis 4, bevorzugt 2 bis 3.

Solche Polyesterpolyole dienen als Vorprodukt für Polyurethane, Polyesterharze und Polyacrylate und werden so in zahlreichen Anwendungen eingesetzt.

Gefordert werden für diese Anwendungen vor allem farblose bzw. möglichst schwach gefärbte Produkte ohne Eigengeruch und mit hoher Lagerstabilität.

Die Herstellung von Polyesterpolyolen aus mehrwertigen Alkoholen und Dicarbonsäuren unter Auskreisen von Wasser ist allgemein bekannt.

Die Reaktionen laufen herkömmlich unter Katalyse, beispielsweise mit einer Säure, oder nur durch Temperaturerhöhung und gegebenenfalls unter Vakuumbehandlung ab.

Da die Polyesterpolyole von mehrwertigen Alkoholen aufgrund ihrer hohen Siedepunkte in der Regel nicht destillativ gereinigt werden können, verbleiben Nebenprodukte im Zielester und beeinflussen die Weiterverarbeitung und/oder die Qualität sowohl des Zielesters als auch der Folgeprodukte.

Die Aufgabe der vorliegenden Erfindung bestand darin, ein wirtschaftliches Verfahren zu entwickeln, das die Herstellung von Polyesterpolyolen mehrwertiger Alkohole in hoher Reinheit und hoher Ausbeute auf einfache Weise und ohne zusätzliche Hilfsstoffe im technischen Maßstab ermöglicht.

Gefunden wurde nun ein Verfahren zur Herstellung von Polyesterpolyolen durch Umsetzung von mehrwertigen Alkoholen mit mindestens einer Dicarbonsäure und/oder einem Derivat, beispielsweise einem Anhydrid einer Dicarbonsäure in gegebenenfalls einem Lösungsmittel unter Abtrennung des Reaktionswassers, wobei der eingesetzte mehrwertige Alkohol einen Formaldehydacetalgehalt unter 500 ppm aufweist.

### Das gefundene Verfahren hat folgenden entscheidenden Vorteil:

Das Endprodukt ist deutlich weniger gefärbt, Farbzahlschwankungen zwischen unterschiedlichen Produktionskampagnen treten nicht auf, und ein Ausdünsten oder Fogging von Inhaltsstoffen im Produkt kann vermieden werden.

Als mehrwertige Alkohole können beispielsweise eingesetzt werden Trimethylolbutan, Trimethylolpropan, Trimethylolethan, Neopentylglykol, Pentaerythrit, 2-Ethyl-1,3-Propandiol, 2-Methyl-1,3-Propandiol, Glycerin, Ditrimethylolpropan, Dipentaerythrit, Bisphenol A, Bisphenol F, Bisphenol B, Bisphenol S, 2,2-Bis(4-hydroxycyclohexyl)propan, 1,1-, 1,2-, 1,3- und 1,4-Cyclohexandimethanol, 1,2-, 1,3- oder 1,4-Cyclohexandiol, Sorbitol, Mannitol, Diglycerol, Erythritol oder Xylit.

Bevorzugt werden im erfindungsgemäßen Verfahren solche mehrwertigen Alkohole eingesetzt, die durch Umsetzung eines Aldehyds mit Formaldehyd und anschließender Überführung der Aldehydgruppe in eine Hydroxygruppe erhalten werden.

Dies sind beispielsweise mehrwertige Alkohole der Formel (I) :

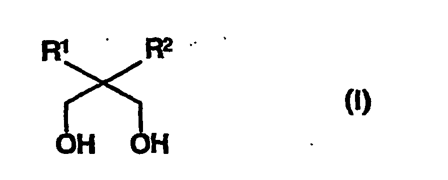

Darin bedeuten
- R¹, R²: unabhängig voneinander Wasserstoff, C₁ - C₁₀-Alkyl, C₁ - C₁₀-Hydroxyalkyl, Carboxyl oder C₁ - C₄-Alkoxycarbonyl, bevorzugt Wasserstoff, Hydroxymethyl und C₁ - C₁₀-Alkyl und besonders bevorzugt Hydroxymethyl und C₁ - C₁₀-Alkyl.

Dabei können die Alkylreste jeweils geradkettig oder verzweigt sein.

Beispiele für R¹ und R² sind Wasserstoff, Methyl, Ethyl, iso-Propyl, n-Propyl, n-Butyl, iso-Butyl, sek-Butyl, tert-Butyl, n-Pentyl, n-Hexyl, n-Heptyl, n-Octyl, n-Decyl, Hydroxymethyl, Carboxyl, Methoxycarbonyl, Ethoxycarbonyl oder n-Butoxycarbonyl, bevorzugt Wasserstoff, Hydroxymethyl, Methyl und Ethyl, besonders bevorzugt Hydroxymethyl, Methyl und Ethyl.

Beispiele für mehrwertige Alkohole der Formel (I) sind Trimethylolbutan, Trimethylolpropan, Trimethylolethan, Neopentylglykol, Pentaerythrit, 2-Ethyl-1,3-Propandiol, 2-Methyl-1,3-Propandiol, 1,3-Propandiol, Dimethylolpropionsäure, Dimethylolpropionsäuremethylester, Dimethylolpropionsäureethylester, Dimethylolbuttersäure, Dimethylolbuttersäuremethylester oder Dimethylolbuttersäureethylester, bevorzugt sind Neopentylglykol, Trimethylolpropan, Pentaerythrit und Dimethylolpropionsäure, besonders bevorzugt sind Neopentylglykol, Trimethylolpropan und Pentaerythrit, ganz besonders bevorzugt Trimethylolpropan und Pentaerythrit und insbesondere Trimethylolpropan.

Solche mehrwertigen Alkohole der Formel (I) sind beispielsweise erhältlich durch Umsetzung eines Aldehydes der Formel (II), 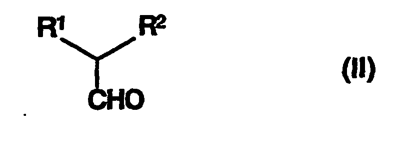 worin R¹ und R² die obigen Bedeutungen haben, mit Formaldehyd und anschließender Überführung der Aldehydgruppe in eine Hydroxygruppe.

Zur Herstellung der erfindungsgemäßen Polyesterpolyole können als Säuren Carbonsäuren mit mindestens zwei Säuregruppen, vorzugsweise aliphatische oder aromatische Dicarbonsäuren, insbesondere solche mit 2 bis 12 Kohlenstoffatomen, verwendet werden. Als Dicarbonsäuren kommen beispielsweise in Betracht: Adipinsäure, Bernsteinsäure, Glutarsäure, Korksäure, Azelainsäure, Sebacinsäure, Decandicarbonsäure, Maleinsäure, Fumarsäure, dimere und/oder trimere Fettsäuren und vorzugsweise Adipinsäure, Phthalsäure, Isophthalsäure, Terephthalsäure und die isomeren Naphthalindicarbonsäuren. Die Dicarbonsäuren können dabei sowohl einzeln als auch im Gemisch untereinander verwendet werden. Anstelle der freien Dicarbonsäuren können auch die entsprechenden Dicarbonsäurederivate, wie z.B. Dicarbonsäureester von Alkoholen mit 1 bis 4 Kohlenstoffatomen oder Dicarbonsäureanhydride eingesetzt werden. Vorzugsweise verwendet werden Dicarbonsäuregemische aus Bernstein-, Glutar- und Adipinsäure in Mengenverhältnissen von beispielsweise 20 bis 35 : 35 bis 50 : 20 bis 32 Gew.-Teilen und Adipinsäure und insbesondere Mischungen aus Phthalsäure und/oder Phthalsäureanhydrid und Adipinsäure, Mischungen aus Phthalsäure(anhydrid), Isophthalsäure und Adipinsäure oder Dicarbonsäuregemische aus Bernstein-, Glutar- und Adipinsäure und Mischungen aus Terephthalsäure und Adipinsäure oder Dicarbonsäuregemische aus Bernstein-, Glutar- und Adipinsäure. Für den Einsatz in Polyurethan-Hartschaumstoffen werden vorzugsweise aromatische Carbonsäuren oder Gemische, die aromatische Carbonsäuren enthalten, eingesetzt. Weiterhin bevorzugt werden Fettsäuren und deren Derivate, auch dimere und/oder trimere Fettsäuren einzeln oder im Gemisch mit eingesetzt. Polyesteralkohole auf Basis von langkettigen Carbonsäuren, insbesondere Fettsäuren, können bevorzugt zur Herstellung von Alkydharzen, die zu Lacken weiterverarbeitet werden können, eingesetzt werden.

Die Alkohole der allgemeinen Formel (I) können im Gemisch mit weiteren mehrwertigen Alkoholen, vorzugsweise Diolen, mit 2 bis 12 Kohlenstoffatomen, vorzugsweise 2 bis 6 Kohlenstoffatomen eingesetzt werden. Beispiele für zwei- und mehrwertige Alkohole, insbesondere Diole sind: Ethandiol, Diethylenglykol, 1,2- bzw. 1,3-Propandiol, Dipropylenglykol, 1,4-Butandiol, 1,5-Pentandiol, 1,6-Hexandiol, 1,10-Decandiol, Glycerin und Trimethylolpropan. Vorzugsweise verwendet werden Ethandiol, Diethylenglykol, 1,4-Butandiol, 1,5-Pentandiol, 1,6-Hexandiol oder Mischungen aus mindestens zwei der genannten Diole, insbesondere Mischungen aus 1,4-Butandiol, 1,5-Pentandiol und 1,6-Hexandiol. Eingesetzt werden können ferner Polyesterpolyole aus Lactonen, z.B. ε-Caprolacton oder Hydroxycarbonsäuren, z.B. ω-Hydroxycapronsäure und Hydroxybenzoesäuren.

Gegebenenfalls können im Gemisch mit den mehrfunktionellen Alkoholen und Carbonsäuren zur Einstellung der Funktionalität auch monofunktionelle Alkohole und/oder Carbonsäuren verwendet werden. Beispiele für monofunktionelle Carbonsäuren sind monomere Fettsäuren, beispielsweise Ölsäure oder Rizinolsäure. Beispiele für monomere Alkohole sind aliphatische Alkohole mit 1 bis 15, vorzugsweise 2 bis 10 Kohlenstoffatomen, beispielsweise Hexanol, Octanol, Nonanol oder Decanol.

Die Alkohole der allgemeinen Formel (I) werden, insbesondere wenn sie eine Funktionalität von größer 2 aufweisen, vorzugsweise in einer Menge von maximal 50 Gew.-%, bezogen auf das Gewicht des Polyesteralkohols, eingesetzt, da es ansonsten zu unerwünschten Vernetzungen und damit verbunden zu einer hohen Viskosität der Polyesteralkohole kommt.

Zur Herstellung der Polyesterpolyole können die organischen, z.B. aliphatischen und vorzugsweise aromatischen Carbonsäuren, und Mischungen aus aromatischen und aliphatischen Polycarbonsäuren und/oder -derivate und mehrwertigen Alkohole katalysatorfrei oder vorzugsweise in Gegenwart von Veresterungskatalysatoren, zweckmäßigerweise in einer Atmosphäre aus Inertgasen, wie z.B. Stickstoff, Kohlenmonoxid, Helium, Argon u.a. in der Schmelze bei Temperaturen von 150 bis 250°C, vorzugsweise 180 bis 220°C gegebenenfalls unter vermindertem Druck bis zu der gewünschten Säurezahl, die vorteilhafterweise kleiner als 10, vorzugsweise kleiner als 2 ist, polykondensiert werden. Zur Herstellung der Polyesterpolyole werden die organischen Polycarbonsäuren und/oder -derivate und mehrwertigen Alkohole vorteilhafterweise im Molverhältnis von 1:1 bis 1,8, vorzugsweise 1:1,05 bis 1,2 polykondensiert.

Als Katalysatoren können saure Katalysatoren, wie Toluolsulfonsäuren, vorzugsweise metallorganische Verbindungen, insbesondere solche auf Basis von Titan oder Zinn, wie Titantetrabutylat oder Zinn-(II)-octoat, eingesetzt werden.

Es ist auch möglich, den mehrwertigen Alkohol zunächst mit Alkylenoxiden zu einem Polyetheralkohol umzusetzen und diesen mit Carbonsäuren zu verestern. Ebenso ist es möglich, den nach dem erfindungsgemäßen Verfahren hergestellten Polyesteralkohol mit einem Alkoxylierungskatalysator zu versetzen, um Alkylenoxid anzulagern. Die so erhaltenen Polyetheresterole zeichnen sich durch gute Verträglichkeit mit Polyetherolen und Polyesterolen aus und können vorzugsweise zur Herstellung von Polyurethanen eingesetzt werden.

Die mehrwertigen Alkohole (I) werden in technischem Maßstab durch Kondensation von Formaldehyd mit höheren, CH-aciden Aldehyden (II) oder mit Wasser und Acrolein bzw. 2-Alkylacroleinen erhalten. Dabei unterscheidet man bei dieser Reaktion zwischen zwei prinzipiellen Durchführungsvarianten der Überführung der Aldehydgruppe in eine Hydroxygruppe, die im folgenden an der Herstellung von Trimethylolpropan illustriert, jedoch keinesfalls darauf beschränkt sein soll.

Zum einen ist dies das sogenannte Cannizzaro-Verfahren, das wiederum unterteilt wird in das anorganische und das organische Cannizzaro-Verfahren. Bei der anorganischen Variante setzt man einen Überschuß an Formaldehyd mit dem entsprechenden Aldehyd (II), also n-Butyraldehyd, in Gegenwart von stöchiometrischen Mengen einer anorganischen Base wie NaOH oder Ca(OH)₂ um. Das in der ersten Stufe gebildete Dimethylolbutanal reagiert in der zweiten Stufe mit dem überschüssigen Formaldehyd in einer Disproportionierungsreaktion zu Trimethylolpropan und dem Formiat der entsprechenden Base, also etwa zu Natrium- oder Kalziumformiat. Der Anfall dieser Salze stellt einen Nachteil dar, da sie schwierig vom Reaktionsprodukt abzutrennen sind, und außerdem ein Äquivalent Formaldehyd verloren geht.

Bei dem organischen Cannizzaro-Verfabren wird anstelle einer anorganischen Base ein tertiäres Alkylamin eingesetzt. Damit lassen sich höhere Ausbeuten erzielen als mit einer anorganischen Base. Es fällt als unerwünschtes Nebenprodukt Trialkylammoniumformiat an. Somit geht auch hier ein Äquivalent Formaldehyd verloren.

Die Nachteile des Cannizzaro-Verfahrens werden bei dem sogenannten Hydrierverfahren vermieden. Dabei bringt man Formaldehyd mit dem entsprechenden Aldehyd (II) in Gegenwart von katalytischen Mengen eines Amins zur Reaktion. Damit wird erreicht, daß die Reaktion im wesentlichen auf der Stufe des alkylolierten Aldehyds stoppt. Nach Abtrennung des Formaldehyds wird das Reaktionsgemisch, das neben dem erwähnten alkylolierten Aldehyd noch geringe Mengen des entsprechenden mehrwertigen Alkohols und von Acetalen der gebildeten Alkohole enthält, einer katalytischen Hydrierung unterworfen, bei der der gewünschte mehrwertige Alkohol erhalten wird.

Ein besonders effektives Verfahren zur Herstellung von durch Kondensation von Aldehyden mit Formaldehyd erhältlichen mehrwertigen Alkoholen wird dabei in der WO 98/28253 beschrieben. Hohe Ausbeuten, verbunden mit dem Anfallen lediglich geringer Mengen an Koppelprodukten, werden mit diesem Verfahren ermöglicht. Es wird dabei so verfahren, dass der höhere Aldehyd mit der 2- bis 8-fachen Menge Formaldehyd in Gegenwart eines tertiären Amins umgesetzt wird, und man das so erhaltene Reaktionsgemisch in zwei Lösungen auftrennt, wobei eine Lösung das erwähnte vollständig methylolierte Alkanal und die andere Lösung nicht umgesetztes Ausgangsprodukt aufweist. Diese letzte Lösung wird in die Reaktion zurückgeführt. Die Auftrennung erfolgt durch Destillation oder einfaches Abtrennen der wässrigen von der organischen Phase. Die das Produkt enthaltende Lösung wird einer katalytischen und/oder thermischen Behandlung unterworfen, um nicht-vollständig alkylolierte Alkanale in die gewünschten vollständig methylolierten Verbindungen zu überführen. Hierbei entstandenes Nebenprodukt wird durch Destillation abgetrennt, und der so erhaltene Sumpf wird der katalytischen Hydrierung, die zu den mehrwertigen Alkoholen führt, unterworfen.

Besonders bevorzugt werden bei dem erfindungsgemäßen Verfahren zur Herstellung von Polyesterpolyolen mehrwertige Alkohole der Formel (I) eingesetzt, die nach dem Hydrierverfahren erhalten worden sind, also durch Umsetzung eines Aldehyds der Formel (II) mit Formaldehyd und anschließender Überführung der Aldehydgruppe in eine Hydroxygruppe durch katalytische Hydrierung erhalten worden sind, besonders bevorzugt solche, die nach dem in der WO 98/28253 beschriebenen Verfahren erhalten worden sind.

Erfindungsgemäß wesentlich ist, daß der Formaldehydacetalgehalt in dem eingesetzten mehrwertigen Alkohol unter 500 Gew.-ppm und bevorzugt unter 400 Gew.-ppm beträgt.

Als Formaldehydacetale (Formale) werden dabei solche cyclischen oder aliphatischen Verbindungen verstanden, die das Strukturelement

-O-CH₂-O- (Formel III)

beinhalten.

Dies können solche Halb- oder Vollacetale sein, die sich von Hauptkomponenten und Verunreinigungen, sowie Neben-, Zwischen- oder Folgeprodukten des Reaktionsgemisches ableiten.

Dabei kann es sich beispielsweise um folgende Formaldehydacetale der Formel (IV) handeln:

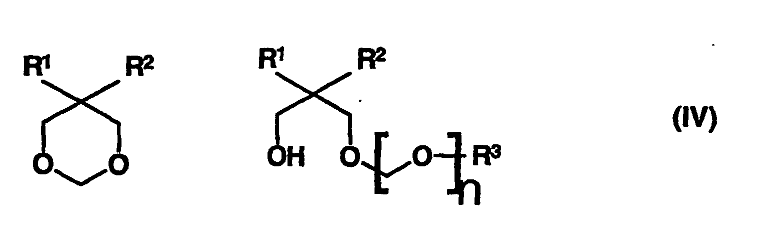

Darin haben R¹ und R² die oben angeführten Bedeutungen, ferner bedeuten
- R³: geradkettiges oder verzweigtes C₁ - C₁₀-, bevorzugt C₁ - C₈- und besonders bevorzugt C₁ - C₅-Alkyl, geradkettiges oder verzweigtes C₁ - C₁₀-, bevorzugt C₁ - C₈-und besonders bevorzugt C₁ - C₆-Hydroxyalkyl oder Wasserstoff und
- n: eine ganze Zahl von 1 bis 4, bevorzugt von 1 bis 3 und besonders bevorzugt von 1 bis 2.

Beispiele für R³ sind Wasserstoff, Methyl, Ethyl, n-Propyl, n-Butyl, 2-Methylpropyl, 2-Methylbutyl, 2-Ethyl-3-hydroxypropyl, 2-Methyl-3-hydroxypropyl, 2,2-Bis(hydroxymethyl)butyl, 2,2-Bis(hydroxymethyl)propyl, 2,2-Dimethyl-3-hydroxypropyl, 3-Hydroxypropyl, 3-Hydroxy-2-(hydroxymethyl)propyl oder 3-Hydroxy-2,2-bis (hydroxymethyl) propyl.

Bevorzugt handelt es sich um folgende Formaldehydacetale:

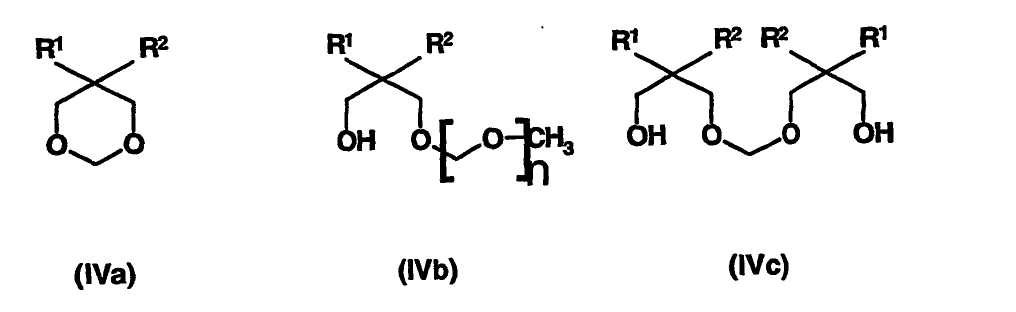

Darin haben R¹, R² und n die oben genannten Bedeutungen.

Besonders bevorzugt handelt es sich um die Formaldehydacetale IVa, IVb (n = 1), IVb (n = 2) und IVc.

Dabei bilden sich die Methanolacetale aus Methanol, der in Formaldehyd in der Regel zu einem niedrigen Anteil enthalten ist, beziehungsweise sich während der Herstellung durch Cannizzaro-Reaktion von Formaldehyd in geringen Mengen bildet.

Typische Formaldehydacetale sind beispielsweise für den Fall der Synthese des dreiwertigen Alkohols Trimethylolpropan (TMP) aus Formaldehyd und n-Butyraldehyd in Gegenwart katalytischer Mengen an Trialkylamin die Formaldehydacetale IVa, IVb (n = 1), IVb (n = 2) und IVc, worin jeweils R¹ Ethyl und R² Hydroxymethyl ist, welche im Rohprodukt des Hydrierverfahren zu 0,05 bis 10 Gew.-% enthalten sein können.

Der Formaldehydacetalgehalt berechnet sich als Summe des Molgewichtsanteils von Formaldehydäquivalenten an dem jeweiligen Formaldehydacetal, multipliziert mit dessen analytisch gefundenen Gewichtsanteil am Reaktionsgemisch.

So berechnet sich beispielsweise der Formaldehydacetalgehalt für ein Trimethylolpropangemisch (R¹ = Ethyl, R² = Hydroxymethyl), das die Komponenten (IVa), (IVb, mit n = 1 und n = 2) sowie (IVc) enthält wie folgt:$\text{Formaldehydacetalgehalt [Gew.-%] =} \text{Gew.-% (IVa) ×} \frac{\text{30 g/mol}}{\text{146 g/mol}} \text{+ Gew.-% (IVb, n = 1) ×} \frac{\text{30 g/mol}}{\text{178 g/mol}} \text{+} \text{Gew.-% (IVb, n = 2) ×} \frac{\text{2x30 g/mol}}{\text{208 g/mol}} \text{+ Gew.-% (IVc) ×} \frac{\text{30 g/mol}}{\text{280 g/mol}}$

Um den entsprechenden Formaldehydacetalgehalt in Gew.-ppm zu erhalten, ist dieser Wert mit 10.000 zu multiplizieren.

Der Gehalt der jeweiligen Komponenten ist nach dem Fachmann an sich bekannten analytischen Methoden bestimmbar, beispielsweise per Gaschromatographie oder HPLC. Die Identifizierung der jeweiligen Komponenten ist beispielsweise durch Kopplung der genannten analytischen Methoden mit Massenspektrometrie möglich.

Es ist erfindungsgemäß nicht relevant, wie ein solch niedriger Formaldehydacetalgehalt im mehrwertigen Alkohol erzielt wird.

In US 6 096 905 ist ein Verfahren offenbart, bei dem eine Formaldehydacetale enthaltende Zusammensetzung mit einem stark sauren Katalysator bei 30 bis 300°C 1/2 bis 8 Stunden lang behandelt wird.

GB-A 1 290 036 beschreibt ein Verfahren, bei dem eine nach dem anorganischen Cannizzaro-Verfahren erhaltene TMP-Rohlösung, mit einem Kationentauscher behandelt wird.

Ein bevorzugtes Verfahren, den Formaldehydacetalgehalt in einem mehrwertigen Alkohol zu verringern, besteht darin, dass man den mehrwertigen Alkohol nach seiner Herstellung durch Destillation reinigt, anschließend einer Temperung unterwirft und anschließend erneut reinigt, vorzugsweise durch Destillation, wie es in der deutschen Anmeldung mit dem Aktenzeichen 100 29 055.8 und dem Anmeldedatum 13. Juni 2000 der BASF AG oder in der internationalen Anmeldung mit dem Titel "Verfahren zum Entfernen von formaldehydhaltigen Acetalen aus mehrwertigen Alkoholen durch Tempern" der BASF AG beschrieben ist.

Wenn mehrwertige Alkohole in einen solchen Temperschritt eingesetzt werden, können besonders gute Ergebnisse beim Einsatz von Alkohollösungen mit einem Gehalt von mehr als 60 %, bevorzugt > 75 %, besonders bevorzugt > 90 %, ganz besonders bevorzugt > 95 % und insbesondere > 98 % erzielt werden. Als weitere Bestandteile können die Alkohollösungen beispielsweise Lösungsmittel, wie z.B. Wasser, Methanol, Ethanol oder n-Butanol, sowie bei der Herstellung des mehrwertigen Alkohols auftretende Nebenprodukte enthalten, bevorzugt in Mengen unter 10 Gew.-%, besonders bevorzugt in Mengen unter 5 Gew.-% und ganz besonders bevorzugt unter 2 Gew.-%.

Dieses Verfahren kann dabei zur Verringerung des Formaldehydacetalgehalts in mehrwertigen Alkoholen, bevorzugt solchen Alkoholen der Formel (I) und insbesondere Trimethylolpropan, jegiicher Herkunft verwendet werden. Es können Chargen eingesetzt werden, die dem organischen oder dem anorganischen Cannizzaro-Verfahren entstammen. Die besten Ergebnisse wurden erhalten, wenn in dem der Formaldehydacetalreduktion dienenden Verfahren Alkohole eingesetzt wurden, die aus dem Hydrierverfahren stammen. Es ist dabei in jedem Fall wichtig, dass der Alkohol vorher gereinigt wurde und eine Reinheit aufweist, die in dem oben genannten Bereich liegt.

Sollen mit dem Verfahren Formaldehydacetale aus Rohlösungen mehrwertiger Alkohole, insbesondere Trimethylolpropan mit Produktgehalten von 60 bis 95 Gew.-% entfernt werden, wird bevorzugt das nach dem Hydrierverfahren erhaltene Rohprodukt (der Hydrieraustrag) vor dem Temperschritt einer Entwässerung unterzogen, bei der Wasser und andere Leichtsieder, wie Methanol und Trialkylamin oder Trialkylammoniumformiat, durch Destillation abgetrennt werden.

Damit bei diesem Verfahren die gewünschte Verringerung des Formaldehydacetalgehalts erzielt wird, müssen bestimmte Reaktionsbedingungen eingehalten werden, die in Abhängigkeit von etwa der Art des eingesetzten mehrwertigen Alkohols, den Reinheiten der eingesetzten Produkte, den verwendeten Apparaturen und eventuell weiteren vorhandenen Bestandteilen oder Zusatzstoffen variieren können. Diese Reaktionsbedingungen sind dem Fachmann durch Versuche zugänglich.

Generell wird der Temperschritt bei Temperaturen von 100 bis 300°C, vorzugsweise 160 bis 240°C, Verweilzeiten von 5 min bis 24 h, vorzugsweise 15 min bis 4 h und Drücken von 100 mbar bis 200 bar vorzugsweise 1 bis 10 bar durchgeführt.

Wenn der zu reinigende mehrwertige Alkohol Trimethylolpropan ist, wird der Temperschritt bei Temperaturen von 100 bis 300°C, vorzugsweise 160 bis 240°C, Verweilzeiten von 10 min bis 24 h, vorzugsweise 1 h bis 5 h, besonders bevorzugt 30 min bis 6 h und ganz besonders bevorzugt 45 min bis 4 h, und den vorstehend erwähnten Drücken durchgeführt.

Zur Durchführung des Temperschritts können die üblichen, dem Fachmann bekannten Apparaturen verwendet werden, wobei dieser kontinuierlich oder diskontinuierlich durchgeführt werden kann. Bei der diskontinuierlichen Durchführung (Batch-Fahrweise) wird der Temperschritt vorzugsweise in einem Rührbehälter durchgeführt, bei der kontinuierlichen Fahrweise in einem Rohrreaktor, wobei hier auf eine Sumpf- oder Rieselfahrweise zurückgegriffen werden kann.

Die meistbevorzugte Ausführungsform des Temperschrittes ist das kontinuierliche Durchführen in einem Rohrreaktor in Sumpffahrweise.

Bei allen diesen Durchführungsvarianten kann der Reaktionsbehälter mit den üblichen, einem Fachmann bekannten Schütt-Füllkörpern, beispielsweise Raschig- oder Pall-Ringen oder Packungen, wie beispielsweise Blechpackungen, versehen sein, um eine bessere Durchmischung der Komponenten zu erreichen. Auch können Träger und/oder Katalysatoren in den üblichen Konfektionierungen, beispielsweise Strängen oder Tabletten, vorhanden sein, um die bei dem Temperschritt ablaufenden Reaktionen zu beschleunigen. Beispiele für geeignete Träger/Katalysatoren umfassen TiO₂, Al₂O₃, SiO₂, geträgerte Phosphorsäure (H₃PO₄) und Zeolithe.

In einer Variante des Temperschrittes wird ein geeigneter Zusatzstoff während des Temperschritts der Reaktionslösung zugefügt, um die zur Verringerung der Formaldehydacetale führenden Reaktionen zu beschleunigen und zu erleichtern. Geeignet sind hierfür nicht zu starke und/oder reduzierende Säuren bzw. deren Anhydride oder Ionentauscher, wie in der US 6 096 905 oder GB 1 290 036 beschrieben ist. Beispiele für geeignete Säuren umfassen Phosphorsäure, Phosphorige Säure, Hypophosphorige Säure, Borsäure, Kohlensäure und schweflige Säure. Auch Gase wie beispielsweise CO₂ und SO₂, die in wässriger Lösung sauer reagieren, sind geeignet.

Die als Zusatzstoff zu verwendenden Säuren werden eingesetzt in Mengen von 10 ppm bis 1 Gew.-%, vorzugsweise 100 bis 2000 ppm. Da der eventuell zugegebene Hilfsstoff nach dem Temperschritt von dem formaldehydacetalreduzierten mehrwertigen Alkohol abgetrennt werden muß, ist es bevorzugt, wenn dieser Zusatzstoff gasförmig ist und daher einfach durch Ausgasen der Reaktionsmischung entfernt werden kann.

Weiterhin kann es vorteilhaft sein, den Temperschritt zur Zersetzung der Formaldehydacetale unter einem inerten Gas durchzuführen, beispielsweise Stickstoff, Argon oder Helium, bevorzugt unter Stickstoff.

Ohne an eine Theorie gebunden zu sein wird vermutet, daß Formaldehydacetale durch den Temperschritt in dem durch Destillation vorgereinigten Alkohol in höhersiedende, schwerflüchtige und leichtsiedende Komponenten überführt werden und so leichter destillativ abtrennbar sind.

Der mehrwertige Alkohol mit vermindertem Formaldehydacetalgehalt läßt sich von den entstehenden hochsiedenden schwerflüchtigen Komponenten durch Destillation leicht abtrennen. Dem Temperschritt folgt daher generell eine Destillation. Da die in dem Temperschritt aus den Formaldehydacetalen entstehenden schwerflüchtigen Verbindungen sich im allgemeinen hinsichtlich ihres Siedeverhaltens deutlich von den mehrwertigen Alkoholen unterscheiden, können diese durch einfache, nur eine geringe Trennwirkung aufweisende destillative Maßnahmen bzw. Methoden abgetrennt werden. Oftmals genügen dabei Trenneinheiten mit nur einer Destillationsstufe, beispielsweise Fallfilmverdampfer oder Dünnfilmverdampfer. Gegebenenfalls, insbesondere wenn die Destillation auch der weiteren Reinigung des Produktalkohols dient, werden aufwendigere Trennverfahren bzw. Trennapparaturen zum Einsatz kommen, generell Kolonnen mit mehreren Trennstufen, beispielsweise Füllkörperkolonnen, Glockenbodenkolonnen oder Packungskolonnen.

Bei der Destillation werden die üblichen, einem Fachmann bekannten Bedingungen hinsichtlich Druck und Temperatur eingehalten werden, wobei diese selbstverständlich auch von dem eingesetzten Produktalkohol abhängig sind.

Entsprechend einer weiteren Ausführungsform kann der Temperschritt auch mit der Destillation zusammengefaßt werden. Dabei findet dann die Temperung im Kolonnensumpf der Destillationsvorrichtung statt, in der der mehrwertige Produktalkohol von den bei der Temperung entstehenden schwerflüchtigen Komponenten, sowie gegebenenfalls anderen Verunreinigungen abgetrennt wird. Werden Temperschritt und Destillation in einer Stufe zusammengefaßt, so ist es wichtig, daß die oben dargelegten Reaktionsbedingungen hinsichtlich Druck, Temperatur und insbesondere der Verweilzeit eingehalten werden, um eine ausreichende Zersetzung der Formaldehydacetale zu erreichen. Bei der Kombination von Temper- und Destillationsschritt zu einem einzigen Verfahrensschritt ist der Zusatz von Säure bevorzugt.

Der durch dieses Verfahren erhältliche mehrwertige Alkohol weist im allgemeinen einen Formaldehydacetalgehalt wie oben definiert unter 500 Gew.-ppm auf, bevorzugt unter 400 Gew.-ppm.

Dabei ist es unwesentlich, nach welchem Verfahren der mehrwertige Alkohol erhalten wurde, beispielsweise nach dem Cannizzaro oder dem Hydrierverfahren.

Die nach dem erfindungsgemäßen Verfahren hergestellten Polyesterole können beispielsweise durch Reaktion mit Polyisocyanaten zu Polyurethanen umgesetzt werden.

In dieser Schrift verwendeten ppm- und Prozentangaben beziehen sich, falls nicht anders angegeben, auf Gewichtsprozente und -ppm.

### Beispiele

Herstellung von Polyesterpolyolen für technischen Hartschaum:

### Beispiel I

In einer Laborrührapparatur mit Heizung, Stickstoffeinperlung, Füllkörperkolonne und Destillationseinrichtung wurden 204,1 g Ölsäure, 105,6 g Adipinsäure, 214,1 g Phthalsäureanhydrid und 541,2 g TMP (Formaldehydacetalgehalt 280 ppm, Diformalin- und Methanolgehalt 0,4%) vorgelegt und unter Rühren auf 170°C erhitzt. Nach Entfernung von 60,4 g Reaktionswasser wurde die Temperatur auf 220°C erhöht. Unter weiterer Entfernung des entstehenden Destillats wurde die Reaktion bis zur Säurezahl kleiner 0,5 mg KOH/g geführt.

### Beispiel II und III (Vergleich)

Gemäß Beispiel I wurde die Synthese mit TMP mit einem Formaldehydacetalgehalt von 620 ppm und 1400 ppm wiederholt.

| Versuchs-Nr. | Formaldehydacetalgehalt des eingesetzten TMPs [Gew.-ppm] | Farbzahl des Polyesterpolyols [Iod-Farbzahl] |
|---|---|---|
| I | 280 | 7,5 |
| II | 620 | 8,1 |
| III | 1400 | 8,6 |

## Patentansprüche

1. Verfahren zur Herstellung von Polyesterpolyolen von mehrwertigen Alkoholen durch einfache oder mehrfache Veresterung mindestens einer Dicarbonsäure und/oder mindestens einem Derivat einer Dicarbonsäure mit mehrwertigen Alkoholen und gegebenenfalls unter Zusatz eines Katalysators unter Abtrennung des Reaktionswassers, **dadurch gekennzeichnet, dass** der eingesetzte mehrwertige Alkohol einen Formaldehydacetalgehalt unter 500 ppm aufweist.

2. Verfahren nach Anspruch 1, **dadurch gekennzeichnet, daß** es sich bei dem mehrwertigen Alkohol um einen Alkohol der Formel (I) handelt, 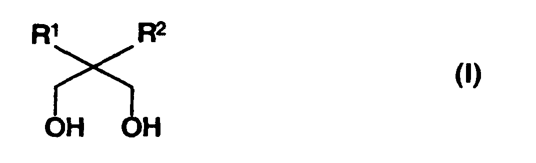 worin R¹ und R² unabhängig voneinander Wasserstoff, C₁ - C₁₀-Alkyl, C₁ - C₁₀-Hydroxyalkyl, Carboxyl oder C₁ - C₄-Alkoxycarbonyl bedeuten.

3. Verfahren nach einem der Ansprüche 1 bis 2, **dadurch gekennzeichnet, daß** es sich bei dem mehrwertigen Alkohol um Trimethylolbutan, Trimethylolpropan, Trimethylolethan, Neopentylglykol, Pentaerythrit, 2-Ethyl-1,3-Propandiol, 2-Methyl-1,3-Propandiol, 1,3-Propandiol, handelt.

4. Verfahren nach Anspruch 2 bis 3, **dadurch gekennzeichnet, daß** der mehrwertige Alkohol durch Umsetzung eines Aldehydes der Formel (II), 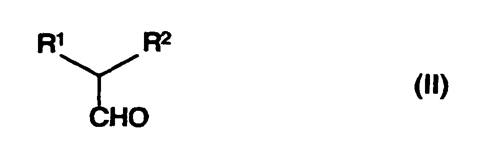 worin R¹ und R² die in Anspruch 2 angegebenen Bedeutungen haben, mit Formaldehyd und anschließender Überführung der Aldehydgruppe in eine Hydroxygruppe durch katalytische Hydrierung erhalten worden ist.

5. Verfahren nach einem der vorstehenden Ansprüche, **dadurch gekennzeichnet, daß** man den mehrwertigen Alkohol (I) nach seiner Herstellung durch Destillation reinigt, anschließend einer Temperung unterwirft und anschließend erneut reinigt.

6. Verfahren nach Anspruch 5, **dadurch gekennzeichnet, daß** man die Temperung bei 100 bis 300°C durchführt.

7. Verfahren nach Anspruch 1, **dadurch gekennzeichnet, daß** als Dicarbonsäuren aliphatische oder aromatische Dicarbonsäuren mit 2 bis 12 Kohlenstoffatomen verwendet werden.

8. Verfahren nach Anspruch 1, **dadurch gekennzeichnet, daß** als Carbonsäuren mit mindestens zwei Säuregruppen Ölsäuren eingesetzt werden.

9. Verfahren nach Anspruch 1, **dadurch gekennzeichnet, daß** im Gemisch mit den Dicarbonsäuren monofunktionelle Carbonsäuren eingesetzt werden.

## Claims

1. A process for preparing polyester polyols of polyhydric alcohols by mono- or polyesterification of at least one dicarboxylic acid and/or at least one derivative of a dicarboxylic acid with polyhydric alcohols, optionally with the addition of a catalyst, while removing the water of reaction, wherein the polyhydric alcohol used has a formaldehyde acetal content of less than 500 ppm.

2. A process as claimed in claim 1, wherein the polyhydric alcohol is an alcohol of the formula (I) 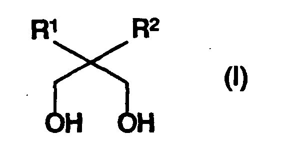 where R¹ and R² are each independently hydrogen, C₁-C₁₀-alkyl, C₁-C₁₀-hydroxyalkyl, carboxyl or C₁-C₄-alkoxycarbonyl.

3. A process as claimed in claim 1 or 2, wherein the polyhydric alcohol is trimethylolbutane, trimethylolpropane, trimethylolethane, neopentyl glycol, pentaerythritol, 2-ethyl-1,3-propanediol, 2-methyl-1,3-propanediol, 1,3-propanediol.

4. A process as claimed in claim 2 or 3, wherein the polyhydric alcohol has been obtained by reacting an aldehyde of the formula (II) 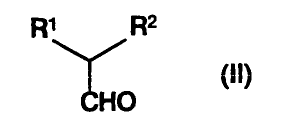 where R¹ and R² are each as defined in claim 2 with formaldehyde and then converting the aldehyde group to a hydroxyl group by catalytic hydrogenation.

5. A process as claimed in any of the preceding claims, wherein the polyhydric alcohol (I), after its preparation, is purified by distillation, then subjected to heat treatment and then purified again.

6. A process as claimed in claim 5, wherein the heat treatment is carried out at from 100 to 300°C.

7. A process as claimed in claim 1, wherein the dicarboxylic acids used are aliphatic or aromatic dicarboxylic acids having from 2 to 12 carbon atoms.

8. A process as claimed in claim 1, wherein the carboxylic acids having at least two acid groups used are oleic acids.

9. A process as claimed in claim 1, wherein monofunctional carboxylic acids are used in a mixture with the dicarboxylic acids.

## Revendications

1. Procédé de préparation de polyesterpolyols d'alcools polyfonctionnels par estérification simple ou multiple d'au moins un acide dicarboxylique et/ou d'au moins un dérivé d'un acide dicarboxylique par des alcools polyfonctionnels et éventuellement avec addition d'un catalyseur et séparation de l'eau réactionnelle, **caractérisé en ce que** l'alcool polyfonctionnel mis en oeuvre présente une teneur en formaldéhyde-acétal inférieure à 500 ppm.

2. Procédé suivant la revendication 1, **caractérisé en ce que**, en ce qui concerne l'alcool polyfonctionnel, il s'agit d'un alcool de la formule (I) : 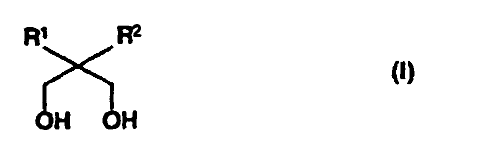 dans laquelle R¹ et R² représentent, indépendamment l'un de l'autre, de l'hydrogène ou un groupe alkyle en C₁-C₁₀, hydroxyalkyle en C₁-C₁₀, carboxyle ou alcoxycarbonyle en C₁-C₄.

3. Procédé suivant l'une des revendications 1 et 2, **caractérisé en ce que**, en ce qui concerne l'alcool polyfonctionnel, il s'agit de triméthylolbutane, de triméthylolpropane, de triméthyloléthane, de néopentylglycol, de pentaérythrite, de 2-éthyl-1,3-propanediol, de 2-méthyl-1,3-propanediol, de 1,3-propanediol.

4. Procédé suivant la revendication 2 ou 3,
**caractérisé en ce que** l'alcool polyfonctionnel a été obtenu par réaction d'un aldéhyde de la formule (II) : 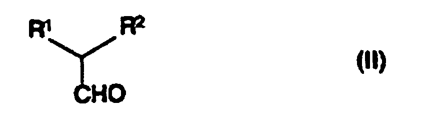 dans laquelle R¹ et R² ont les significations indiquées dans la revendication 2, avec du formaldéhyde et conversion ultérieure du groupe aldéhyde en un groupe hydroxy par hydrogénation catalytique.

5. Procédé suivant l'une des revendications précédentes, **caractérisé en ce qu'**on purifie par distillation l'alcool polyfonctionnel (I) après sa préparation, on le soumet ensuite à un étuvage et on le purifie ensuite à nouveau.

6. Procédé suivant la revendication 5, **caractérisé en ce qu'**on effectue l'étuvage à 100 jusqu'à 300°C.

7. Procédé suivant la revendication 1, **caractérisé en ce que**, comme acides dicarboxyliques, on utilise des acides dicarboxyliques aliphatiques ou aromatiques comportant 2 à 12 atomes de carbone.

8. Procédé suivant la revendication 1, **caractérisé en ce que**, comme acides carboxyliques comportant au moins deux groupes acides, on met en oeuvre des acides oléiques.

9. Procédé suivant la revendication 1, **caractérisé en ce qu'**on met en oeuvre des acides carboxyliques monofonctionnels en mélange avec les acides dicarboxyliques.
